# EUROPEAN PATENT APPLICATION

(11) **EP 4 299 729 A1**
(43) Date of publication of application: **03.01.2024**
(21) Application number: 22760122.6
(22) Date of filing: 25.02.2022
(51) Int. Cl.: C12N 7/00, C07K 14/535, C12N 9/12, C07K 14/705, A61K 35/76, A61P 35/00, C12N 15/62

(54) **ONCOLYTIC VIRUS AND USE THEREOF**

(30) Priority: 26.02.2021 KR 20210026782
(71) Applicant: SillaJen, Inc., Seoul 04525 (KR)
(72) Inventor: OH, Keunhee, Seongnam-si Gyeonggi-do 13615 (KR); LEE, Namhee, Seongnam-si Gyeonggi-do 13555 (KR); JUNG, Byung Jin, Seongnam-si Gyeonggi-do 13504 (KR); LEE, Jun Seung, Yongin-si Gyeonggi-do 16821 (KR); KANG, Kyeong Tae, Seongnam-si Gyeonggi-do 13627 (KR)
(74) Representative: J A Kemp LLP
(86) International application number: PCT/KR2022/002805
(87) International publication number: WO 2022/182206

(57) **Abstract**

The present invention relates to an oncolytic virus and the use thereof, and specifically, an oncolytic virus having suppressed expression of a thymidine kinase (TK) gene and comprising genes encoding granulocyte-macrophage colony-stimulating factor (GM-CSF) and a complement regulatory protein; and the use of such a oncolytic virus . The oncolytic virus of the present invention maintains its efficacy even upon intravenous injection, and thus, it may also be applied to the treatment of various solid carcinomas and metastatic cancers in addition to superficial solid cancers. In addition, the oncolytic virus of the present invention acquires resistance to the attack of the body's complement system by expressing a complement regulatory protein on the surface of the virus, and thus, it is stable in the blood, and it maintains stable oncolytic activity upon intravenous injection, and thus, it may reduce the dose of the virus to minimize the side effects of anti-cancer drugs. Therefore, the oncolytic virus of the present invention may be usefully used for preventing or treating cancer.

## Description

### [Technical Field]

The present invention relates to an oncolytic virus and the use thereof, and specifically, an oncolytic virus having suppressed dexpression of a thymidine kinase (TK) gene and comprising genes encoding granulocyte-macrophage colony-stimulating factor (GM-CSF) and a complement regulatory protein; and the use of such a oncolytic virus.

### [Background Art]

Cancer has become a major cause of death worldwide, and as it imposes enormous personal and social burdens, the importance of developing innovative anti-cancer therapeutic agents is increasing day by day, and accordingly, various approaches are being attempted according to the development of molecular biology.

Oncolytic virus, an anti-cancer therapeutic agent that has recently been in the spotlight, is a virus capable of replication and infectivity, and is a virus that is used for cancer treatment by inserting a specific gene targeting a genetically abnormal site in tumor cells into a wild-type or attenuated virus. In such a genetically engineered oncolytic virus, the spread of the virus through tissue along with cell lysis after viral replication occurs selectively within cancer cells and blood vessels surrounding cancer cells. A representative oncolytic virus is Amgen's Imlygic (talimogene laherparepvec) approved by the U.S. Food and Drug Administration (FDA) as a melanoma therapeutic agent in October 2015.

On the other hand, intravenous injection of pharmaceuticals is an easy way to quickly deliver drugs that cannot be administered orally to the body, and is one of the most preferred forms of administration because it is easy to administer. However, since oncolytic virus preparations are rapidly removed by the body's immune system upon intravenous injection, there is a limitation that their efficacy decreases when administered through intravenous injection. Therefore, in order to increase the probability that the virus reaches the tumor, a method of directly injecting an oncolytic virus preparation into a target tumor rather than intravenous injection is generally used.

The intratumoral injection method may be generally effectively applied to superficial cancers that are easily accessible, such as melanoma, breast cancer and head and neck cancer, but has the disadvantage that the administration of an effective dose in carcinomas such as deep solid cancer is dependent on the technical competence of an operator such as a doctor, and the intratumoral injection procedure is very invasive, and therefore, it is not easy to use it as a repeat treatment method compared to intravenous injection.

Despite the advantages of intravenous injection, the reason why it is difficult to administer oncolytic viruses in the form of intravenous injection is that foreign substances (i.e., viruses) administered into the blood are gradually removed by the human body's defense mechanism to reduce the activity of oncolytic viruses. That is, when viruses move into the body through blood vessels, the human body recognizes them as enemies and activates innate immunity to neutralize and remove them.

When the human body is exposed to bacteria or viruses invading from the outside, the complement system, which is a defense mechanism at the forefront of the innate immune system, acts first. Complement is a protein present in the blood, and serves to facilitate the phagocytosis of macrophages or neutrophils by surrounding the surface of foreign microorganisms. Specifically, when a virus infiltrates into the blood, first, proteins on the surface of the virus combine with complements and aggregate with each other, and complements are activated. Activated complements surround the virus surface to facilitate the phagocytosis of phagocytes (opsonization), and pore the virus surface to lyse the virus. The virus debris lysed in this way is completely removed by phagocytosis of macrophages.

In order to protect surrounding normal cells from damage caused by excessive activation of the complement system, the human body expresses complement regulatory proteins such as CD55, CD46 and CD59 on the cell surface to regulate the excessive action of complement. Among them, CD46 and CD59 proteins inhibit only a part of the complement activation pathway, but CD55 protein (decay accelerating factor (DAF)) is involved in all steps of complement activation and more strongly inhibits complement activation.

A part (about 5 to 20%) of oncolytic viruses propagated in host cells is characterized by the form of "extracellular enveloped virus (EEV) " produced in a manner surrounded by cell membranes of infected cells. This EEV was known to have a long survival period in blood by avoiding complement activation, and its mechanism was found to be due to complement regulatory proteins such as CD55, CD46 and CD59, expressed on the cell membrane of host cells which are surrounded by the viruses and emerges from them. For example, most of the produced oncolytic vaccinia viruses are in the form of "intracellular mature virus (IMV), " which does not express complement regulatory proteins on their surface to greatly reduce their activity by complement. For this reason, there is a need for research and development to improve oncolytic activity upon intravenous injection of an oncolytic virus.

### [Disclosure]

### [Technical Problem]

It is an object of the present invention to provide a novel oncolytic virus, and specifically, an oncolytic virus having suppressed expression of a thymidine kinase (TK) gene and comprising genes encoding granulocyte-macrophage colony-stimulating factor (GM-CSF) and a complement regulatory protein.

It is another object of the present invention to provide a pharmaceutical composition for preventing or treating cancer, comprising the oncolytic virus as an active ingredient.

It is another object of the present invention to provide a genetic construct for insertion into an oncolytic virus, comprising all or part of a gene encoding granulocyte-macrophage colony-stimulating factor (GM-CSF), a gene encoding a transmembrane domain of an oncolytic virus membrane protein and a gene encoding a complement regulatory protein, and operably linked to an early-late promoter and a late promoter for expression.

It is another object of the present invention to provide an anti-cancer adjuvant comprising the oncolytic virus as an active ingredient.

It is another object of the present invention to provide a method of preventing or treating cancer, comprising administering to a subject a composition comprising the oncolytic virus as an active ingredient.

It is another object of the present invention to provide the use of the oncolytic virus or a composition comprising the same for the prevention or treatment of cancer.

It is another object of the present invention to provide the use of the oncolytic virus or a composition comprising the same for the preparation of a drug for preventing or treating cancer.

The technical problems to be achieved according to the technical idea of the invention disclosed in the present specification are not limited to the above-mentioned problems to be solved, and other problems not mentioned will be clearly understood by those skilled in the art from the following description.

### [Technical Solution]

These will be described in detail as follows. On the other hand, each description and embodiment disclosed in the present application may also be applied to each other description and embodiment. That is, all combinations of the various elements disclosed in this application fall within the scope of this application. In addition, it cannot be seen that the scope of the present application is limited by the specific descriptions described below.

In the present invention, in order to improve oncolytic activity upon intravenous injection of the oncolytic viruses presented above, the present inventors have conducted research and development efforts to express complement regulatory proteins, for example, CD55, which has the highest activity, on the surface of "intracellular mature virus (IMV)" to protect oncolytic viruses from complement attack, and have completed the present invention.

As one aspect for achieving the above object of the present invention, the present invention provides an oncolytic virus having suppressed expression of a thymidine kinase (TK) gene and comprising genes encoding granulocyte-macrophage colony-stimulating factor (GM-CSF) and a complement regulatory protein.

In particular, in the present invention, as an example, the CD55 protein was expressed on the surface of the "intracellular mature virus (IMV)" by linking the transmembrane domain in the membrane protein of this oncolytic virus itself to the CD55 gene, and through this, it has resistance to the human body's complement system and maintains stable oncolytic activity upon intravenous injection, as well as reducing the dose of oncolytic virus to minimize side effects of treatment.

The term "oncolytic vaccinia virus" of the present invention may be referred to as "oncolytic virus," which includes a "recombinant virus" engineered through deletion of all or part of endogenous genes or introduction of foreign genes. This oncolytic virus may be vaccinia virus, adenovirus, herpes simplex virus, retrovirus, reovirus, Newcastle disease virus, coxsackie virus, enterovirus, herpes virus, or the like.

The term "thymidine kinase (TK)" of the present invention is an enzyme involved in the biosynthesis of nucleotides. Thymidine kinase encoded by the TK gene may bind phosphoric acid at the gamma (γ) position of ATP to thymidine to produce nucleotides constituting viral DNA. The TK may be, but is not limited to, a sequence such as GenBank: AAR17937.1 or AY313847.1. Specifically, the TK or its gene may be composed of, but is not limited to, the amino acid sequence of GenBank: AAR17937.1 or the base sequence of GenBank: AY313847.1. In addition, the TK or its gene may have about 90%, 91%, 92%, 93%, 94%, 95%, 96%, 97%, 98% or 99% or more homology to the amino acid sequence of GenBank: AAR17937.1 or the base sequence of GenBank: AY313847.1.

The term "granulocyte-macrophage colony-stimulating factor (GM-CSF)" of the present invention is a substance that helps to produce more white blood cells, especially granulocytes, macrophages, and cells that become platelets. It is a cytokine belonging to a class of drugs called hematopoietics (blood-forming agents), and is also known as sargramostim. GM-CSF is produced by many different cell types (for example, activated T-cells, B-cells, macrophages, mast cells, endothelial cells, and fibroblasts) in response to cytokines or immune and inflammatory stimuli. Besides granulocyte-macrophage precursor cells, GM-CSF is also a growth factor for erythrocyte, megakaryocyte and eosinophil precursor cells. For mature hematopoietic cells, GM-CSF is a survival factor for granulocytes, monocytes/macrophages and eosinophils, and activates their effector functions. GM-CSF has also been reported to have a functional role on non-hematopoietic cells. It may induce human endothelial cells to migrate and proliferate.

The GM-CSF may be, but is not limited to, a sequence such as GenBank: M10663.1. Specifically, the GM-CSF gene may be composed of the base sequence of GenBank: M10663.1 or be a part of the sequence, and the GM-CSF gene may have about 90%, 91%, 92%, 93%, 94%, 95%, 96%, 97%, 98% or 99% or more homology to the base sequence of GenBank: M10663.1.

The term "complement regulatory protein" of the present invention is a protein that skillfully regulates the complement activation pathway *in vivo.* It is roughly divided into a serotype (water-soluble) regulatory protein group and a membrane-bound regulatory protein group. The serotype regulatory protein group includes C4b-binding protein, factor H, SGP120, properdin (P), and the like, and the membrane-bound regulatory protein group includes CRI (CD35), CR2 (CD21), CR3 (CD11b/CD18), CR4 (CD11c/CD18), and DAF (CD55), membrane cofactor protein (MCP; CD46), and CD59. Among them, only P acts in the direction of strengthening complement activity, but the others act in the direction of attenuating it. The complement regulatory protein included in the recombinant vaccinia virus of the present invention may be, but is not limited to, CD35, CD21, CD18, CD55, CD46 or CD59, and may specifically be CD55. Specifically, the CD55 gene may be composed of the base sequence of Genbank: NM_000574.3 or be a part of the sequence, and the CD55 gene may have about 90%, 91%, 92%, 93%, 94%, 95%, 96%, 97%, 98% or 99% or more homology to the base sequence of NCBI Reference Sequence: NM_000574.3. More specifically, the CD55 may be composed of the base sequence of SEQ ID NO: 1 or the amino acid sequence of SEQ ID NO: 2.

The term "transmembrane domain" of the present invention is also called a membrane-penetrating region or a membrane-transversing region, and refers to a region that penetrates and traverses the lipid bilayer of a membrane protein. The oncolytic virus according to the present invention may further comprise a gene encoding a transmembrane domain of an oncolytic virus membrane protein. Specifically, the vaccinia virus membrane protein may be D8L, A16L, F9L, G9R, H3L, L1R, A9L, A13L, A21L, A28L, E10R, G3L, H2R, I2L, J5L, L5R or O3L, may comprise all or part of the membrane protein sequence, and is as shown in Table 1 below as an example.

The complement regulatory protein of the present invention may be separately expressed independently of the gene encoding the transmembrane domain, or may be engineered and expressed in a linked fusion form.

**[Table 1]**

| Membrane protein | Classifi cation | Sequence | SEQ ID NO: |
|---|---|---|---|
| D8L | Amino acid | FAIIAIVFVFILTAILFFMSRRYSREKQN | 5 |
| | DNA | ttcgcaattattgccatagtattcgtgtttatacttacc gctattctcttttttatgagtcgacgatattcgcgagaa aaacaaaac | 6 |
| A16L | Amino acid | LGAAITLVVISVIFYFISIYSRPKIKTNDINVRRR | 7 |
| | DNA | ttgggcgcggccataacattggttgtaatatctgttatt ttctattttatatctatttattcgcgtactaaaattaaa acaaatgatataaatgttcgtagacga | 8 |
| F9L | Amino acid | PWFLVGVAIILVIFTVAICSIRRNLALKYRYGTFLYV | 9 |
| | DNA | ccgtggtttctagtgggtgtagcaattattctagttatt tttactgtagctatttgttctattagacgaaatctggct cttaaatacagatacggaacgtttttatacgtt | 10 |
| G9R | Amino acid | LKLHLISLLSLLVIWILIVAI | 11 |
| | DNA | ctaaaattgcatttgatcagtttattatctctcttggta atatggatactaattgtagctatt | 12 |
| H3L | Amino acid | LFDINVIGLIVILFIMFMLIFNVKSKLLWFLTGTFVTAF I | 13 |
| | DNA | ttgtttgatattaatgttataggtttgattgtaattttg tttattatgtttatgctcatctttaacgttaaatctaag ctgttatggttccttacaggaacattcgttaccgcattt | 14 |
| | | atc | |
| L1R | Amino acid | VQFYMIVIGVIILAALFMYYAKRMLFTSTNDKIKLILAN KENVHWTTYMDTFFRTSPMVIATTDMQN | 15 |
| | DNA | gttcagttttatatgattgttatcggtgttataatattg gcagcgttgtttatgtactatgccaagcgtatgttgttc acatccaccaatgataaaatcaaacttattttagccaat aaggaaaacgtccattggactacttacatggacacattc tttagaacttctccgatggttattgctaccacggatatg caaaac | 16 |
| A9L | Amino acid | FVVVRAIASMIMYLVLGIALL | 17 |
| | DNA | ttcgtcgttgttagagccattgcgagcatgataatgtat ttagtattaggtatagcattgctg | 18 |
| A13L | Amino acid | MIGILLLIGICVAVTVAILYA | 19 |
| | DNA | atgattggtattcttttgttgatcggtatttgcgtagca gttaccgtcgccatcctatatgcg | 20 |
| A21L | Amino acid | MITLFLILCYFILIFNIIVPA | 21 |
| | DNA | atgataactttatttttaatcctatgttatttcattctt atttttaatattatagtacctgca | 22 |
| A28L | Amino acid | MNSLSIFFIVVATAAVCLLFI | 23 |
| | DNA | atgaactctctatcaattttttttattgtggtagcgacg gctgcggtgtgtttactttttatc | 24 |
| E10R | Amino acid | AVWTIIFIVLSQAGLDG | 25 |
| | DNA | gccgtatggaccattatttttatagtactttcgcaagcg ggtttagacggc | 26 |
| G3L | Amino acid | MASLLYLILFLLFVCISYYFT | 27 |
| | DNA | atggcatctttattatatcttattttatttttgttattc gtatgtatttcttattattttaca | 28 |
| H2R | Amino acid | TSTLIFFVIILAISALLLWFQ | 29 |
| | DNA | acatcaacacttatattctttgttattatattggcaatt agtgcgctattactctggtttcag | 30 |
| I2L | Amino acid | YWLIIIFFIVLILLLLIYLYL | 31 |
| | DNA | tattggttaattattattttttttatagttcttattcta ctactattgatatatttgtattta | 32 |
| J5L | Amino acid | IIDIKWLPIGLLALAILILAF | 33 |
| | DNA | attatagatatcaaatggcttccgattggattactagcg ttagctattttaatattagcattt | 34 |
| L5R | Amino acid | IVLFEVFVVFILIYVFFRSEL | 35 |
| | DNA | atagttttatttgaagtattcgttgtattcattctaata tatgtattttttagatctgaatta | 36 |
| O3L | Amino acid | LVVIMFFIAFAFCSWLSYSYL | 37 |
| | DNA | ctcgtcgtaattatgttttttatagcgtttgccttctgt agttggctatcatatagctatctg | 38 |

In this case, the complement regulatory protein and the transmembrane domain of the oncolytic virus protein may be designed to include all or part of the gene through a genetic engineering process. Specifically, the transmembrane domain of the membrane protein of a vaccinia virus among oncolytic viruses may be composed of, but is not limited to, the amino acid sequence of SEQ ID NO: 5, SEQ ID NO: 7, SEQ ID NO: 9, SEQ ID NO: 11, SEQ ID NO: 13 or SEQ ID NO: 15, or the base sequence of SEQ ID NO: 6, SEQ ID NO: 8, SEQ ID NO: 10, SEQ ID NO: 12, SEQ ID NO: 14 or SEQ ID NO: 16.

The expression suppression of a gene or the gene inactivation according to the present invention means that the gene is not expressed or only part of the gene is expressed through the deletion of part or all of a gene or the insertion of a foreign gene into a gene so that the activity of the protein encoded by the gene is not shown. A method for deleting the gene or inserting a foreign gene may be performed by methods well known in the art. For example, it may be performed by the method of inserting a foreign gene, disclosed in Molecular Cloning, A Laboratory Manual,Second Edition, by J. Sambrook, E. F. Fritsch and T. Maniatis (2003), Cold Spring Harbor Laboratory Press, Virology Methods Manual, edited by Brian W J Mahy and Hiliar O Kangro (1996) Academic Press and Expression of genes by Vaccinia virus vectors . Current Protocols in Molecular Biology, published by John Wiley and Son (1998), Chapter 16. Specifically, the suppressed expression of the thymidine kinase gene may be due to insertion of a foreign gene into part or all of the thymidine kinase J2R region. More specifically, in one embodiment of the present invention, the foreign gene was inserted using the T-Blunt^{™} PCR Cloning Kit (Solgent, Korea Cat No. SOT01-K020). The GM-CSF and the complement regulatory protein included in the oncolytic virus of the present invention may be expressed under the control of a late-early VACV p7.5 promoter, a vaccinia synthetic early-late promoter (pSEL), a vaccinia synthetic late promoter (pSL), a vaccinia modified H5 (mH5) promoter, a vaccinia short synthetic early-late pS promoter, a pLate promoter, a pC11R promoter, a pF11L promoter, a psFJ1-10 synthetic early promoter, a pHyb synthetic early promoter, any natural vaccinia early promoter, or a late-early optimized (LEO) promoter, respectively, but are not limited thereto. As an example, the GM-CSF may be expressed under the control of the promoter pSEL, and the complement regulatory protein or CD55 may be expressed under the control of the promoter pLate. More specifically, the GM-CSF may be expressed under the control of the promoter pSEL of SEQ ID NO: 3, and the complement regulatory protein may be expressed under the control of the promoter pLate of SEQ ID NO: 4.

The oncolytic virus of the present invention may further comprise a gene capable of increasing cancer therapeutic efficacy. This gene may include, but is not limited to, for example, anti-cancer therapeutic genes, various immune regulators, structural degrading factors such as intratumoral fibers, and the like, and may further include, but is not limited to, for example, IL-1, IL-2, IL-3, IL-4, IL-5, IL-6, IL-7, IL-8, IL-9, IL-10, IL-12, IL-13, IL-14, IL-15, IL-17, IL- 18, IL-21, IL-23, IL-24, interferon-α, interferon-β, interferon-γ, CCL3, CCL5, CXCR4, CXCL9, CXCL10, CXCL11, interleukin superagonist (IL-2 superagonist, IL-15 superagonist), TGF-β blockade, TLR-2 agonist, TLR-3 agonist, TLR-7 agonist, STAT-3 inhibitor, PTENα, p53, p63, p73, adenosine diaminase-2, cancer-specific antigen, cancer-associated antigen, hyaluronidase, collagenase, protease, and the like.

In the present invention, the oncolytic virus includes vaccinia virus, adenovirus, herpes simplex virus, retrovirus, reovirus, Newcastle disease virus, coxsackie virus, enterovirus, herpes virus, and the like, and the vaccinia virus may be, but is not limited to, for example, Western Reserve (WR), New York vaccinia virus (NYVAC), Wyeth, LC16m8, Lister, Copenhagen, Tian Tan, USSR, TashKent, Evans, International Health Division-J (IHD-J), or International Health Division-White (IHD-W) strain.

As another aspect for achieving the above object, the present invention provides a pharmaceutical composition for preventing or treating cancer, comprising the oncolytic virus as an active ingredient.

The term "active ingredient" of the present invention refers to an ingredient that can exhibit the desired activity alone or exhibit activity in combination with a carrier having no activity itself.

The term "cancer (or tumor)" of the present invention includes all without distinction between primary cancer and metastatic cancer. In the present invention, the cancer may be solid cancer or hematologic malignancy. The solid cancer may be any one selected from the group consisting of, but is not limited to, lung cancer, colorectal cancer, prostate cancer, thyroid cancer, breast cancer, brain cancer, head and neck cancer, esophageal cancer, skin cancer, thymus cancer, stomach cancer, colon cancer, liver cancer, ovarian cancer, uterine cancer, bladder cancer, rectal cancer, gallbladder cancer, biliary tract cancer, pancreatic cancer, renal cancer, osteosarcoma, sarcoma, chondrosarcoma, and combinations thereof. The hematologic malignancy may be any one selected from the group consisting of, but not limited to, lymphoma, leukemia, multiple myeloma, and combinations thereof.

The term "metastatic cancer" of the present invention refers to cancer caused by cancer cells leaving a primary organ and moving to other organs and proliferating. The metastatic cancer may include, but is not limited to, both one in which cancer tissue grows from a primary cancer and directly invades surrounding organs, and one in which distant metastasis occurs along blood vessels or lymphatic vessels to other distant organs.

The term "prevention" of the present invention refers to all actions that inhibit or delay the development of cancer through administration of the pharmaceutical composition of the present invention, and the term "treatment" of the present invention refers to all actions that improve, alleviate or change beneficially cancer by administering the pharmaceutical composition of the present invention.

The specific dosage of the pharmaceutical composition comprising the oncolytic virus of the present invention may be variously selected by a person skilled in the art depending on factors such as the formulation method, the condition and body weight of the patient, the sex and age of the patient, the severity of the disease, the form of the drug, the route and duration of administration, the rate of the excretion and the response sensitivity, and the dosage and frequency do not limit the scope of the present invention in any way. Specifically, the pharmaceutical composition of the present invention may comprise, but is not limited to, 10⁵ to about 10¹³ pfu (plaque forming units) of oncolytic virus.

The term "pharmaceutical composition" of the present invention refers to those prepared for the purpose of preventing or treating a disease, and may be formulated and used in various forms according to conventional methods, respectively. For example, depending on the route of administration, it may be formulated in the form of oral formulations such as powders, granules, tablets, capsules, suspensions, emulsions and syrups, and may be formulated and used in the form of external preparations and sterile injectable solutions. Specifically, the route of administration may be any appropriate route including topical route, oral route, intravenous route, intramuscular route, and direct absorption through mucosal tissue, and two or more routes may also be used in combination. An example of a combination of two or more routes is a case where two or more formulations of drugs are combined according to the route of administration, for example, a case where one drug is firstly administered through an intravenous route and the other drug is secondly administered through a topical route.

The pharmaceutical composition of the present invention may be for intratumoral, intravascular, intramuscular or intraperitoneal administration, and may be for intravenous or arterial administration as an example.

The pharmaceutical composition of the present invention may be prepared in the form of a pharmaceutical composition for treating or preventing cancer, further comprising an appropriate carrier, excipient or diluent commonly used in the preparation of pharmaceutical compositions, wherein the carrier may include a non-naturally occurring carrier. Specifically, the pharmaceutical composition may be formulated and used in the form of oral formulations such as powders, granules, tablets, capsules, suspensions, emulsions, syrups and aerosols, external preparations, suppositories and sterile injectable solutions according to conventional methods, respectively. In the present invention, the carrier, excipient and diluent that may be included in the pharmaceutical composition may include lactose, dextrose, sucrose, sorbitol, mannitol, xylitol, erythritol, maltitol, starch, gum acacia, alginate, gelatin, calcium phosphate, calcium silicate, cellulose, methyl cellulose, microcrystalline cellulose, polyvinyl pyrrolidone, water, methylhydroxybenzoate, propylhydroxybenzoate, talc, magnesium stearate, and mineral oil. It is prepared using diluents or excipients, such as a filler, an extender, a binder, a wetting agent, a disintegrating agent, and a surfactant, which are usually used when formulated. Solid formulations for oral administration include tablets, pills, powders, granules, capsules, and the like, and such solid formulations are prepared by mixing one or more excipients, such as starch, calcium carbonate, sucrose or lactose, gelatin, and the like. In addition to simple excipients, lubricants such as magnesium stearate and talc are also used. Liquid formulations for oral administration include suspensions, solutions for internal use, emulsions, syrups, and the like, and may comprise various excipients, such as wetting agents, sweeteners, flavoring agents, preservatives, and the like, in addition to commonly used simple diluents such as water and liquid paraffin. Formulations for parenteral administration include a sterile aqueous solution, a non-aqueous solution, a suspension, an emulsion, a lyophilized formulation, and a suppository. As the non-aqueous solutions and suspensions, propylene glycol, polyethylene glycol, vegetable oils such as olive oil, injectable esters such as ethyl oleate, and so on may be used. As a base for suppositories, witepsol, macrogol, Tween 61, cacao butter, laurin butter, glycerogelatin, and so on may be used.

The specific formulation of the pharmaceutical composition is known in the art, and reference may be made to, for example, Remington's Pharmaceutical Sciences (19th ed., 1995), and the like. This document is considered as part of the present specification.

The pharmaceutical composition of the present invention may be administered to mammal animals such as mice, dogs, cats, cows, horses, pigs, and humans through various routes, and human may be preferred. Any mode of administration may be expected, and may be administered by, but is not limited to, for example, oral, intravenous, arterial, intramuscular or subcutaneous injection.

For example, the oncolytic virus of the present invention has resistance to the human body' s complement system and maintains stable oncolytic activity upon intravenous injection, as well as reducing the dose of oncolytic virus to maximize the therapeutic efficacy, thereby exhibiting sufficient therapeutic efficacy even with intravenous administration.

As another aspect for achieving the above object, the present invention provides a genetic construct for insertion into an oncolytic virus, comprising all or part of a gene encoding granulocyte-macrophage colony-stimulating factor (GM-CSF), a gene encoding a transmembrane domain of a vaccinia virus membrane protein and a gene encoding a complement regulatory protein, and operably linked to an early-late promoter and a late promoter for expression; and a vector containing the same.

In the present invention, the genetic construct may be for insertion into an inactivated thymidine kinase gene region of the oncolytic virus.

In the present invention, the inactivated thymidine kinase gene region includes a region in which all or part of the thymidine kinase gene is deleted.

In the present invention, the genetic construct includes one located between the J1R region and the J3R region in the oncolytic virus.

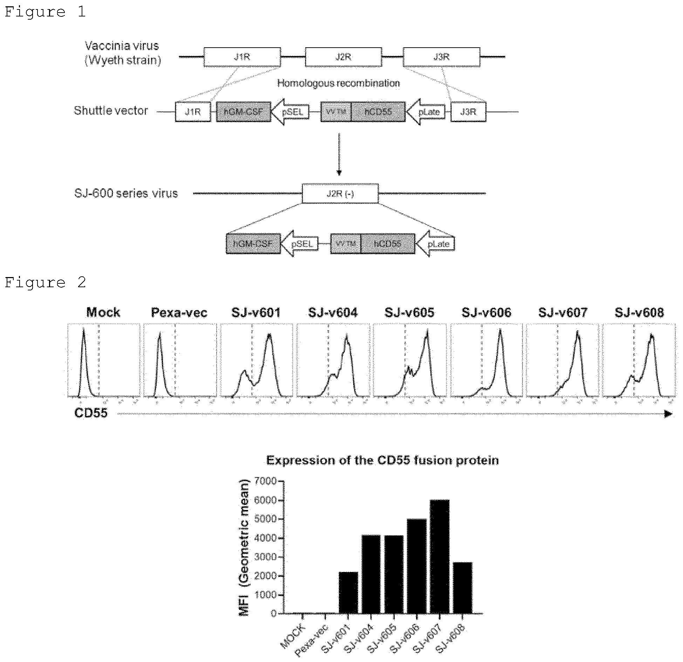

In the present invention, the genetic construct may include, but is not limited to, one illustrated in Figure 1, and may further comprise various promoters and regulatory sequences for regulating gene expression in an operably linked form.

The terms "granulocyte-macrophage colony-stimulating factor (GM-CSF)", "transmembrane domain" and "complement regulatory protein" of the present invention are as described above.

In the present invention, the genetic construct is for insertion into all or part of the thymidine kinase of vaccinia virus, and specifically, is for insertion into all or part of the thymidine kinase J2R region.

As another aspect for achieving the above object, the present invention provides an anti-cancer adjuvant comprising the recombinant vaccinia virus as an active ingredient.

The anti-cancer adjuvant refers to any form for increasing the anti-cancer effect of an anti-cancer drug or suppressing or improving the side effects of an anti-cancer drug. The anti-cancer adjuvant of the present invention may be administered in combination with various types of anti-cancer drugs or anti-cancer adjuvants, and even if the anti-cancer drug is administered at a lower level than the dose of a conventional anti-cancer drug when administered in combination, it may exhibit an equivalent level of anti-cancer therapeutic effect, and thus, safer anti-cancer treatment may be performed.

The anti-cancer adjuvant may be administered through any general route, as long as it can reach a target tissue. The anti-cancer adjuvant of the present invention may be administered, but is not limited to, intraperitoneally, intravenously, intramuscularly, subcutaneously, intrapulmonaryly or intrarectally, as desired. In addition, the anti-cancer adjuvant may be administered by any device capable of transporting an active substance to a target cell.

For the anti-cancer adjuvant of the present invention, the anti-cancer adjuvant may be preferably formulated by including one or more pharmaceutically acceptable carriers in addition to the active ingredient, for administration. The carrier, excipient or diluent that may be included in the anti-cancer therapeutic adjuvant of the present invention may include, but is not limited to, lactose, dextrose, sucrose, sorbitol, mannitol, xylitol, erythritol, maltitol, starch, gum acacia, alginate, gelatin, calcium phosphate, calcium silicate, cellulose, methyl cellulose, microcrystalline cellulose, polyvinyl pyrrolidone, water, methylhydroxybenzoate, propylhydroxybenzoate, talc, magnesium stearate, and mineral oil.

The anti-cancer adjuvant of the present invention may be a formulation for parenteral administration, and the description of the formulation is replaced by a description of the formulation of the pharmaceutical composition.

In addition, any anti-cancer adjuvant disclosed in the technical field may be applied to the present invention without particular limitation.

In one embodiment of the present invention, the anti-cancer drug may be one or more selected from, but is not limited to, chemotherapeutic agents, biologic therapeutic agents, radiotherapy, immunotherapy, hormone therapeutic agents, anti-vascular therapeutic agents, cryotherapeutic agents, and toxin therapeutic agents.

As another aspect for achieving the above object, the present invention provides a method of preventing or treating cancer, comprising administering to a subject a composition comprising the recombinant vaccinia virus as an active ingredient.

The terms "cancer," "prevention" and "treatment" of the present invention are as described above.

The subject may be a mammal animal, and specifically, may be, but is not limited to, a human, cow, sheep, goat, horse, pig, dog, cat, rabbit, rat, mouse, fish, bird, and the like.

The composition comprising the recombinant vaccinia virus as an active ingredient may be appropriately administered by a person skilled in the art depending to the age, sex and body weight of the patient, the severity of the disease, and the route of administration. The administration may be once a day or several times a day, and may be repeatedly administered at an appropriate cycle.

The dosage of the composition comprising the recombinant vaccinia virus as an active ingredient varies depending on the condition and body weight of the subject, the severity of the disease, the form of the drug, and the route and duration of administration, and may be appropriately selected by a person skilled in the art. Specifically, it may be to administer 10⁵ to about 10¹³ pfu (plaque forming units) to the subject, and may include various numerical values or ranges between the above ranges.

In the method of treating cancer of the present invention, the composition may be administered through any general route, as long as it can reach a target tissue. The composition of the present invention may be administered, but is not particularly limited to, through a route such as oral administration or rectal administration, and in some cases, it may be administered through other routes depending on the purpose.

In the present invention, the method of treating cancer includes a treatment method through combined administration with a conventionally known anti-cancer drug and anti-cancer therapy.

Another aspect of the present invention for achieving the above objects provides the use of the oncolytic virus or a composition comprising the same for the prevention or treatment of cancer.

Another aspect of the present invention for achieving the above object provides the oncolytic virus or a composition comprising the same for the preparation of a drug for preventing or treating cancer.

### [Advantageous Effects]

The oncolytic virus of the present invention maintains its efficacy even upon intravenous injection, and thus, it may also be applied to the treatment of various solid carcinomas, hematologic malignancies and metastatic cancers in addition to superficial solid cancers. In addition, the oncolytic virus of the present invention has resistance to the body's complement system by expressing a complement regulatory protein, and especially maintains stable oncolytic activity upon intravenous injection, thereby reducing the dose of the virus to minimize the side effects of anti-cancer drugs. Therefore, the oncolytic virus of the present invention may be usefully used for preventing or treating cancer.

### [Description of Drawings]

Figure 1 is a schematic diagram of a recombinant vaccinia virus, which is the oncolytic virus of the present invention in which the TK gene is deleted and CD55 fused with GM-CSF and the transmembrane domain is introduced.
Figure 2 shows the results of confirming by FACS whether CD55 is expressed in osteosarcoma cells infected with the recombinant vaccinia viruses (SJ-v601 and SJ-v604 to SJ-v608) of the present invention.
Figure 3 shows the results of confirming by Western blotting whether the recombinant vaccinia viruses (SJ-v601 and SJ-v604 to SJ-v608) of the present invention expresses CD55.
Figure 4 shows the results of confirming the survival rate of the recombinant vaccinia viruses (SJ-v601 and SJ-v604 to SJ-v608) of the present invention in 20% active human serum.
Figure 5 shows the results of confirming the recovery of the recombinant vaccinia viruses (SJ-v601 and SJ-v604 to SJ-v608) of the present invention in 20% active human serum.
Figure 6 shows the results of confirming the survival rate of JX-594 and SJ-v607 in 20% and 50% active human serums.
Figure 7 shows the results of confirming the anti-tumor efficacy of JX-594 and SJ-v607 through changes in tumor volume in the A549 lung cancer xenograft model.
Figure 8 shows the results of confirming the change in body weight over time after administration of JX-594 and SJ-v607 to the A549 lung cancer xenograft model.
Figure 9 shows the results of confirming the anti-tumor efficacy of JX-594 and SJ-v607 through changes in tumor volume in the HCT-116 colorectal cancer xenograft model.
Figure 10 shows the results of confirming the change in body weight over time after administration of JX-594 and SJ-v607 to the HCT-116 colorectal cancer xenograft model.

### [Best Mode]

Hereinafter, the configuration and effects of the present invention will be described in more detail through examples . These examples are only for illustrating the present invention, and the scope of the present invention is not limited thereto.

The present inventors constructed a vector into which human GM-CSF and human CD55 genes were inserted, and prepared a recombinant vaccinia virus in which the expression of the above genes was induced by homologous recombination of the vector and vaccinia virus to confirm the characteristics as an anti-cancer substance.

### Preparative Example 1: Preparation of TK-deleted recombinant vaccinia virus SJ-v601 expressing GM-CSF and CD55

As shown in Figure 1, the J2R region of the thymidine kinase region was completely deleted in the Wyeth strain among vaccinia viruses, and CD55 and GM-CSF were inserted at this site. In addition, in order to express the CD55 on the viral outer membrane, the gene from which the GPI anchor region of hCD55 was deleted was fused with the transmembrane domain and cell membrane domain of the vaccinia virus membrane protein, and pLate was used as a promoter for expression. The specific process is as follows:

### 1. Construction of a plasmid vector inducing expression of hGM-CSF and hCD55 genes

To delete the vaccinia virus J2R region, the J1R and J3R genes, which are the left and right flanking regions of the J2R region, were cloned into the T-blunt^{™} vector (Solgent) with the NEBuilder^{®} HiFi DNA Assembly Cloning Kit (NEW ENGLAND BioLabs, Catalog No. M5520A) . J1R and J3R DNAs were used by PCR amplification of corresponding sites of JX-594 (Pexastimogene Devacirepvec, Pexa-Vec). The J1R is identical to a region from amino acid positions 1 to 154 of the J1R (Protein id = AAR17936.1) of the vaccinia virus Acambis2000 strain, but amino acid position 118 among them is substituted from alanine to serine. J3R (Protein id = AAR17938.1) is a region from amino acid positions 1 to 145. hGM-CSF (GenBank: M10663.1) expressed with the vaccinia virus synthetic early-late promoter, the complement regulatory protein hCD55 gene (GenBank: NM_000574.3) expressed with the vaccinia virus late promoter, and the transmembrane domain of the vaccinia virus D8L were cloned into this vector again to complete a shuttle vector. The gene sequence and amino acid sequence of hCD55 used are shown in SEQ ID NOs: 1 and 2, respectively. The transmembrane domain regions of hGM-CSF and D8L were amplified by PCR in JX-594, and hCD55 was amplified by PCR from a vector cloned into pCMV3 plasmid (Sinobio, Catalog No. HG10101-UT). GM-CSF (GenBank: M10663.1) is a region from amino acid positions 1 to 145 and is expressed with a synthetic early-late promoter (aaaaattgaaattttattttttttttttggaatataaata; SEQ ID NO: 3; pSEL; used by PCR amplification from Pexa-Vec). hCD55 (GenBank: NM_000574.3) is a region from amino acid positions 1 to 352 in which the GPI anchor region was deleted, and the promoter for expression is a synthetic late promoter (ttttttttttttttttttttggcatataaata; SEQ ID NO: 4; pLate; synthesized by Macrogen) . The 3'-end of the hCD55 gene was linked to the transmembrane domain region of vaccinia virus D8L to express CD55 on the viral outer membrane, and information and base sequences of the transmembrane domain region of D8L used are shown in Tables 2 and 3.

**[Table 2]**

| Virus | Membrane protein | Transmembrane domain region | | |
|---|---|---|---|---|
| | | Position | Transmembrane | Intravirion |
| SJ-v601 | D8L | 276-304 | 276-294 | 295-304 |

**[Table 3]**

| Membrane protein | | Sequence | SEQ ID NO: |
|---|---|---|---|
| D8L | Amino acid | FAIIAIVFVFILTAILFFMSRRYSREKQN | SEQ ID NO: 5 |
| | DNA | ttcgcaattattgccatagtattcgtgtttatacttacc gctattctcttttttatgagtcgacgatattcgcgagaa aaacaaaac | SEQ ID NO: 6 |

### 2. Preparation of the recombinant vaccinia virus SJ-v601 by homologous recombination

The recombinant vaccinia virus SJ-v601 was prepared by homologous recombination of the wild-type vaccinia virus Wyeth strain and the previously constructed plasmid vector in which the expression of hGM-CSF and hCD55 genes was induced.

The wild-type Wyeth strain was prepared by inserting the J2R region gene of the vaccinia virus Western Reserve (WR) strain (ATCC, Catalog No. VR-1354) into the inactivated J2R region (TK region) of JX-594 (Pexastimogene Devacirepvec, Pexa-Vec). J2R (Protein id = YP_232976.1) of the Western Reserve strain is a region from amino acid positions 1 to 178. The J2R region gene of the Western Reserve strain was amplified by PCR, and the wild-type Wyeth strain was completed by homologous recombination of the PCR product and JX-594.

For recombination of SJ-v601, 143B osteosarcoma cells (Creative Bioarray) were infected with the wild-type Wyeth strain, and transfected with plasmid vectors expressing hGM-CSF and hCD55. In order to select the recombinant virus SJ-v601 plaques in which the TK region was deleted, the cell lysate in which 143B osteosarcoma cells were infected with vaccinia virus and transfected with the plasmid vector was treated with the TK deletion selection reagent 5-bromo-2'-deoxyuridine (BrdU), and cultured. The selected recombinant virus plaques were further subcultured twice in 143B cells again to obtain purified single clone SJ-v601. The base sequence of the recombination site of SJ-v601 was finally confirmed through sequencing.

### Preparative Example 2: Preparation of TK-deleted recombinant vaccinia viruses SJ-v604 to SJ-v608 expressing hGM-CSF and CD55

A plasmid vector used for recombination was constructed in the same manner as in Example 1 above, except that the transmembrane domain of D8L was used in Example 1 above, and the transmembrane domains of other membrane proteins were used to prepared SJ-v604 to SJ-v608. Information on the transmembrane domain used to prepare SJ-v604 to SJ-v608 is shown in the table below, and the sequence of the transmembrane domain of each membrane protein is also shown in the table below.

**[Table 4]**

| Virus | Membrane protein | Transmembrane domain region | | |
|---|---|---|---|---|
| | | Position | Transmembrane | Intravirion |
| SJ-v604 | A16L | 343-377 | 343-363 | 364-377 |
| SJ-v605 | F9L | 176-212 | 176-196 | 197-212 |
| SJ-v606 | G9R | 320-340 | 320-340 | N/A |
| SJ-v607 | H3L | 285-324 | 285-305 | 306-324 |
| SJ-v608 | L1R | 184-250 | 184-204 | 205-250 |

**[Table 5]**

| Membrane protein | | Sequence | SEQ ID NO: |
|---|---|---|---|
| A16L | Amino acid | LGAAITLVVISVIFYFISIYSRPKIKTNDINVRRR | SEQ ID NO: 7 |
| | DNA | ttgggcgcggccataacattggttgtaatatctgttatt ttctattttatatctatttattcgcgtactaaaattaaa acaaatgatataaatgttcgtagacga | SEQ ID NO: 8 |
| F9L | Amino acid | PWFLVGVAIILVIFTVAICSIRRNLALKYRYGTFLYV | SEQ ID NO: 9 |
| | DNA | ccgtggtttctagtgggtgtagcaattattctagttatt tttactgtagctatttgttctattagacgaaatctggct cttaaatacagatacggaacgtttttatacgtt | SEQ ID NO: 10 |
| G9R | Amino acid | LKLHLISLLSLLVIWILIVAI | SEQ ID NO: 11 |
| | DNA | ctaaaattgcatttgatcagtttattatctctcttggta atatggatactaattgtagctatt | SEQ ID NO: 12 |
| H3L | Amino acid | LFDINVIGLIVILFIMFMLIFNVKSKLLWFLTGTFVTAF I | SEQ ID NO: 13 |
| | DNA | ttgtttgatattaatgttataggtttgattgtaattttg tttattatgtttatgctcatctttaacgttaaatctaag ctgttatggttccttacaggaacattcgttaccgcattt atc | SEQ ID NO: 14 |
| L1R | Amino acid | VQFYMIVIGVIILAALFMYYAKRMLFTSTNDKIKLILAN KENVHWTTYMDTFFRTSPMVIATTDMQN | SEQ ID NO: 15 |
| | DNA | gttcagttttatatgattgttatcggtgttataatattg gcagcgttgtttatgtactatgccaagcgtatgttgttc acatccaccaatgataaaatcaaacttattttagccaat aaggaaaacgtccattggactacttacatggacacattc tttagaacttctccgatggttattgctaccacggatatg caaaac | SEQ ID NO: 16 |

Finally, the structures of SJ-v601 and SJ-v604 to SJ-v608 prepared in Preparative Examples 1 and 2 above are shown in the table below.

**[Table 6]**

| Virus | Structure |
|---|---|
| SJ-v601 | wyeth △TK-hGMCSF-hCD55-D8L |
| SJ-v604 | wyeth △TK-hGMCSF-hCD55-A16L |
| SJ-v605 | wyeth △TK-hGMCSF-hCD55-F9L |
| SJ-v606 | wyeth △TK-hGMCSF-hCD55-G9R |
| SJ-v607 | wyeth △TK-hGMCSF-hCD55-H3L |
| SJ-v608 | wyeth △TK-hGMCSF-hCD55-L1R |

### Example 1: Confirmation of CD55 expression of SJ-v601 and SJ-v604 to SJ-v608 in osteosarcoma cells

Osteosarcoma cells U-2 OS were infected with the recombinant vaccinia viruses SJ-v601 and SJ-v604 to SJ-v608 prepared in Preparation Examples 1 and 2 above, and after 16 hours, FACS analysis was performed to confirm whether CD55 was expressed. HeLa cells were used as a positive control, and TK-deleted JX-594 (Pexa-vec) expressing GM-CSF and Lac-Z was used as a negative control.

Specifically, U-2 OS was seeded in a 6-well plate at 4 x 10⁵ cells per well and cultured overnight. The next day, recombinant vaccinia viruses SJ-v601 and SJ-v604 to SJ-v608 were diluted to 0.5 plaque forming unit (PFU) per cell in DMEM medium containing 2.5% heat-inativated FBS. U-2 OS cells inoculated the previous day were infected with the diluted virus at 37°C for 16 hours. After 16 hours, infected cells were harvested, washed with PBS, and then treated with Fixation/Permeabilization solution (BD, Catalog No. 565388) to fix and permeabilize the cells. Thereafter, the cells were stained with an anti-hCD55 antibody (BioLegend, Catalog No. 311312) coupled with an APC fluorescent factor, and the expression of APC was measured by FACS (BD LSR Fortessa), and then analyzed with Flowjo software.

As a result, as shown in Figure 2, it was confirmed that CD55 was expressed in cells infected with SJ-v601 and SJ-v604 to SJ-v608 viruses.

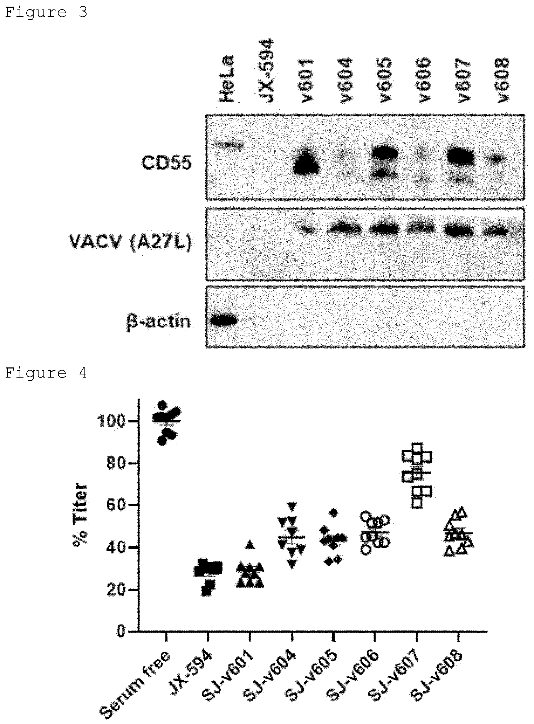

In addition, as a result of performing Western blotting by lysing the virus in the form of purified intracellular mature virus (IMV), as shown in Figure 3, it was confirmed that CD55 was expressed in SJ-v601 and SJ-v604 to SJ-v608 viruses themselves.

Specifically, HeLa cells were infected with the recombinant vaccinia viruses SJ-v601 and SJ-v604 to SJ-v608, respectively, for about 40-48 hours to proliferate, and the infected cells were centrifuged to remove the culture broth, and then cell lysis buffer was added to lyse the cells. The cell lysate was treated with Benzonase to remove cell-derived DNA, and filtered through a cellulose acetate filter to remove cell by-products. The filtrate was concentrated using a 36% sucrose cushion centrifugation method, and purified recombinant vaccinia virus was prepared.

In order to isolate proteins present in the purified virus, the centrifuged virus precipitate was lysed with RIPA lysis buffer (Thermo scientific, Catalog No. 89900), and the amount of protein was quantified by BCA protein assay (Thermo scientific, Catalog No. 23227). Proteins obtained from each virus were diluted in loading buffer (Biosesang, Catalog No. S2002), prepared to be loaded at 1 ug per lane, and electrophoresis was performed on SDS-PAGE gel (BIO-RAD, Catalog No. 4561083) . The electrophoresed gel was again transferred to a nitrocellulose membrane (BIO-RAD, Catalog No. 1704270). Membrane was blocked with 5% skim milk (Difco, Catalog No. 232100) for 1 hour to detect the desired protein, and treated with an anti-hCD55 antibody (SantaCruz, Catalog No. sc-51733), an anti-vaccinia virus A27 antibody (beiResources, Catalog No. NR-627) and an anti-β-actin antibody (invitrogen, Catalog No. MA5-15739) overnight at 4°C. The next day, the membrane was washed 3-4 times with 1X TSBT, treated with secondary antibody coupled with HRP, and then treated with hydrogen peroxide and luminol substrate, and chemiluminescence was confirmed with a detector.

### Example 2: Confirmation of serum stability of SJ-v601 and SJ-v604 to SJ-v608 in serum

In order to confirm whether CD55 expressed on the viral envelope regulates complements present in blood, stability in serum was measured by *in vitro* POC. Specifically, a plaque assay was performed by mixing 20% active human serum with a complement function with the virus.

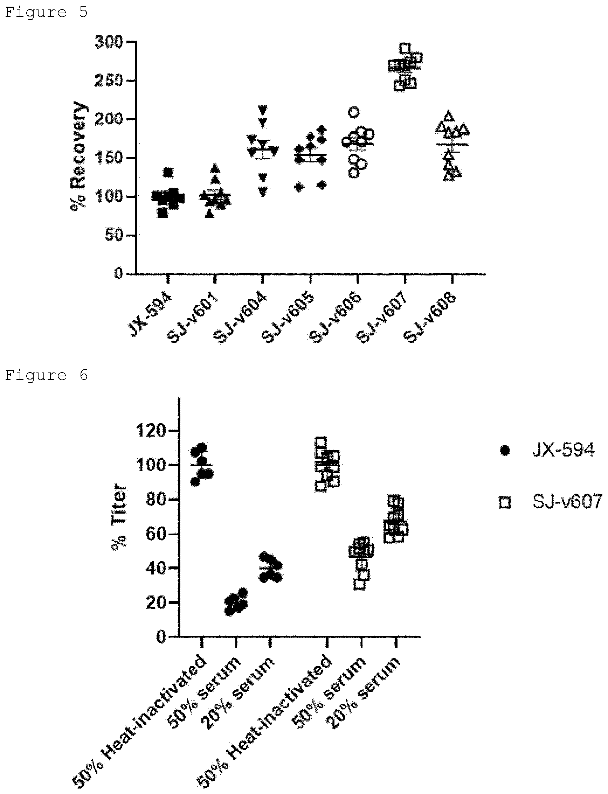

Specifically, U-2 OS osteosarcoma cells were seeded in a 12-well tissue culture plate at 2.5 x 10⁵ cells per well and cultured overnight. The next day, when the cells reached almost 100% confluency, the recombinant vaccinia viruses SJ-v601 and SJ-v604 to SJ-v608 were diluted to about 100 PFU per well in serum-free DMEM medium. Human serum was added to the diluted virus again to 20% of the total volume, and treated for 2 hours in the plate in which the U-2 OS cells were cultured. As a control group, serum-free DMEM medium was used instead of the human serum. After 2 hours, the virus was removed from the 12-well plate and treated with 1.5% carboxymethyl cellulose solution. The 12-well plate was cultured at 37°C for 72 hours, and plaques formed were stained with 0.1% crystal violet solution and counted. When the number of plaques generated in the case of serum-free is 100%, the ratio of the number of plaques generated from each recombinant virus treated with 20% human serum and cultured is shown in Figure 4. The results are as shown in Figure 5 when the same results were compared with the number of plaques generated by the control virus JX-594 not expressing hCD55 as 100%.

As a result, as shown in Figures 4 and 5, it was confirmed that the survival rate of the SJ-v600 series virus according to the present invention was higher compared to that of the control group JX-594. For example, JX-594 remained only 28% of plaque, whereas SJ-v607, which showed the highest survival rate, exhibited a survival rate of 76%.

### Example 3: Confirmation of serum stability of SJ-v607 in serum

In order to confirm whether CD55 expressed on the viral envelope regulates complements present in blood, stability in serum was measured by *in vitro* POC. Specifically, a plaque assay was performed by mixing 20% to 50% active human serums with a complement function with the virus.

U-2 OS osteosarcoma cells were seeded in a 12-well tissue culture plate at 2.5 x 10⁵ cells per well and cultured overnight. The next day, when the cells reached almost 100% confluency, the recombinant vaccinia viruses SJ-v601 and SJ-v604 to SJ-v608 were diluted to about 100 PFU per well in serum-free DMEM medium. Human serum was added to the diluted virus again to 20% to 50% of the total volume, and treated for 2 hours in the plate in which the U-2 OS cells were cultured. As a control group, 50% human serum in which complement in serum was inactivated by heat-inactivation was used. After 2 hours, the virus was removed from the 12-well plate and treated with 1.5% carboxymethyl cellulose solution. The 12-well plate was cultured at 37°C for 72 hours, and plaques formed were stained with 0.1% crystal violet solution and counted. When the number of plaques generated in the 50% heat-inactivated human serum control group is 100%, the ratio of the number of plaques generated from each recombinant virus treated with 20% to 50% human serums and cultured is shown in Figure 6.

As a result of measuring the survival rates of the control group JX-594 and the recombinant vaccinia virus SJ-v607 of the present invention in 20% and 50% serums, it was confirmed that the survival rate of SJ-v607 was higher, and the survival rate in 20% serum was higher than that in 50% serum (Figure 6).

### Example 4: Evaluation of anti-tumor efficacy in lung cancer transplant model

A xenograft mouse tumor model was established by injecting 2 x 10⁶ human lung cancer cells A549 subcutaneously into the flank of NSG immunodeficient mice. When the tumor volume reached about 120 mm³, SJ-v607 or the control group JX-549 were administered as a single intravenous injection, and the tumor growth inhibitory efficacies were compared. SJ-v607 and JX-549 were administered at low (1 x 10⁶ pfu) or high (5 x 10⁶ pfu) concentrations, respectively.

Tumor volumes were measured in the long axis and short axis using a caliper 2-3 times a week in all groups. Tumor volume was calculated as (short axis x short axis x long axis) ÷ 2. In addition, in order to evaluate the body weight of the mouse, the weight was measured at each time point of measuring the tumor volume. The experiment was terminated when the tumor volume reached 1,500 to 2,000 mm³ or more.

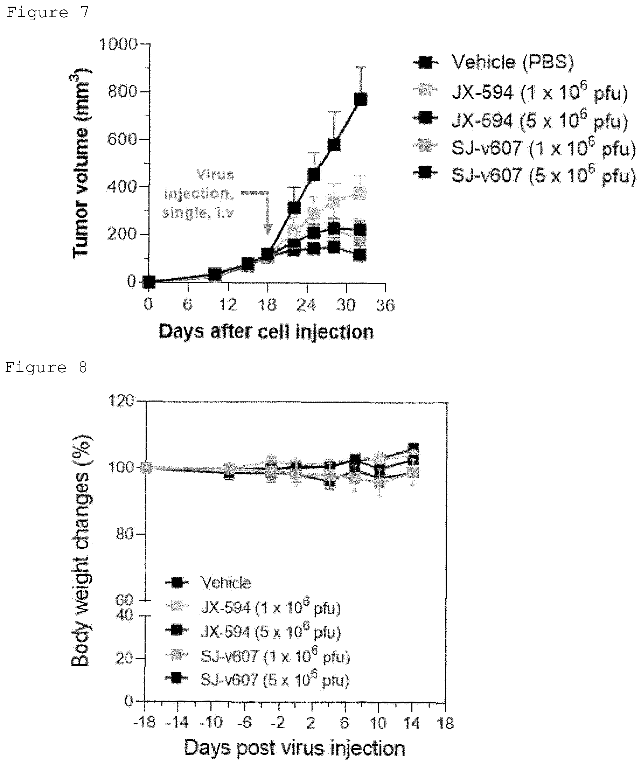

As a result, as shown in Figure 7, it was confirmed that when the recombinant vaccinia virus SJ-v607 of the present invention was administered, the tumor volume was smaller compared to that of the control group JX-594, and the tumor volume decreased after about 24 days (6 days post virus injection), unlike JX-594; and when SJ-v607 was administered at a high concentration (5 x 10⁶ pfu), the tumor volume was smaller compared to that at a low concentration (1 x 10⁶ pfu), and thus, the recombinant vaccinia virus of the present invention had a remarkably excellent anti-tumor effect. In addition, when the oncolytic virus was administered, there was a temporary weight loss compared to the control group not administered with anti-cancer drugs, but it was soon recovered and the body weight was maintained in the normal range according to the decrease in tumor volume (Figure 8).

### Example 5: Evaluation of anti-tumor efficacy in colorectal cancer transplant model

A xenograft mouse tumor model was established by injecting 5 x 10⁵ human colorectal cancer cells HCT-116 subcutaneously into the flank of NSG immunodeficient mice. When the tumor volume reached about 120 mm³, 5 x 10⁶ pfu of SJ-v607 or the control group JX-549 were intravenously injected (single administration), and the tumor growth inhibitory efficacies were compared. Tumor volumes were measured in the long axis and short axis using a caliper 2-3 times a week in all groups. Tumor volume was calculated as (short axis x short axis x long axis) ÷ 2. In addition, in order to evaluate the body weight of the mouse, the weight was measured at each time point of measuring the tumor volume. The experiment was terminated when the tumor volume reached 1, 500 to 2, 000 mm³ or more.

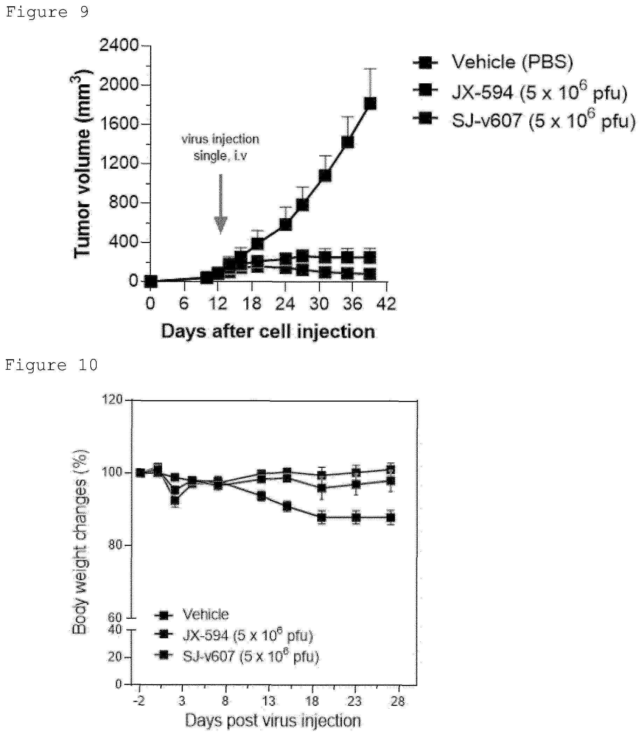

As a result, as shown in Figure 9, it was confirmed that when the recombinant vaccinia virus SJ-v607 of the present invention was administered, the tumor volume was smaller in the entire period after administration compared to the control group JX-594, and thus, it had a remarkably excellent anti-tumor effect. In addition, when the oncolytic virus was administered, there was a temporary weight loss compared to the control group not administered with anti-cancer drugs, but it was soon recovered and the body weight was maintained in the normal range according to the decrease in tumor volume (Figure 10).

## Claims

1. An oncolytic virus having suppressed expression of a thymidine kinase (TK) gene and comprising genes encoding granulocyte-macrophage colony-stimulating factor (GM-CSF) and a complement regulatory protein.

2. The oncolytic virus according to claim 1, wherein the suppressed expression of the thymidine kinase gene is due to deletion of part or all of the gene, or insertion of a foreign gene into the gene.

3. The oncolytic virus according to claim 2, wherein a foreign gene is inserted into part or all of the thymidine kinase J2R region.

4. The oncolytic virus according to claim 1, wherein the complement regulatory protein is CD35, CD21, CD18, CD55, CD46, or CD59.

5. The oncolytic virus according to claim 1, further comprising a gene encoding a transmembrane domain of an oncolytic virus membrane protein.

6. The oncolytic virus according to claim 5, wherein the gene encoding the transmembrane domain of the oncolytic virus membrane protein is fused with a gene encoding the complement regulatory protein.

7. The oncolytic virus according to claim 5, wherein the oncolytic virus membrane protein is D8L, A16L, F9L, G9R, H3L, L1R, A9L, A13L, A21L, A28L, E10R, G3L, H2R, I2L, J5L, L5R, or O3L.

8. The oncolytic virus according to claim 1, wherein the complement regulatory protein is CD55 composed of the sequence of SEQ ID NO: 1.

9. The oncolytic virus according to claim 1, wherein the GM-CSF and the complement regulatory protein are expressed under the control of a late-early VACV p7.5 promoter, a vaccinia synthetic early-late promoter (pSEL), a vaccinia synthetic late promoter (pSL), a vaccinia modified H5 (mH5) promoter, a vaccinia short synthetic early-late pS promoter, a pLate promoter, a pC11R promoter, a pF11L promoter, a psFJ1-10 synthetic early promoter, a pHyb synthetic early promoter, any natural vaccinia early promoter, or a late-early optimized (LEO) promoter, respectively.

10. The oncolytic virus according to claim 1, wherein the oncolytic virus is vaccinia virus, adenovirus, herpes simplex virus, retrovirus, reovirus, Newcastle disease virus, coxsackie virus, enterovirus, or herpes virus.

11. The oncolytic virus according to claim 10, wherein the vaccinia virus is Western Reserve (WR), New York vaccinia virus (NYVAC), Wyeth, LC16m8, Lister, Copenhagen, Tian Tan, USSR, TashKent, Evans, International Health Division-J (IHD-J), or International Health Division-White (IHD-W) strain.

12. A pharmaceutical composition for preventing or treating cancer, comprising the oncolytic virus according to any one of claims 1 to 11 as an active ingredient.

13. The pharmaceutical composition according to claim 12, wherein the cancer is solid cancer or hematologic malignancy.

14. The pharmaceutical composition according to claim 13, wherein the solid cancer is any one selected from the group consisting of lung cancer, colorectal cancer, prostate cancer, thyroid cancer, breast cancer, brain cancer, head and neck cancer, esophageal cancer, skin cancer, thymus cancer, stomach cancer, colon cancer, liver cancer, ovarian cancer, uterine cancer, bladder cancer, rectal cancer, gallbladder cancer, biliary tract cancer, pancreatic cancer, renal cancer, osteosarcoma, sarcoma, chondrosarcoma, and combinations thereof.

15. The pharmaceutical composition according to claim 13, wherein the hematologic malignancy is any one selected from the group consisting of lymphoma, leukemia, multiple myeloma, and combinations thereof.

16. The pharmaceutical composition according to claim 12, wherein the composition is for intratumoral, intravascular, intramuscular or intraperitoneal administration.

17. The pharmaceutical composition according to claim 16, wherein the composition is for intravenous or arterial administration.

18. A genetic construct for insertion into an oncolytic virus, comprising all or part of a gene encoding granulocyte-macrophage colony-stimulating factor (GM-CSF), a gene encoding a transmembrane domain of a vaccinia virus membrane protein and a gene encoding a complement regulatory protein, and operably linked to an early-late promoter and a late promoter for expression.

19. The genetic construct according to claim 18, wherein the genetic construct is for insertion into an inactivated thymidine kinase gene region of the oncolytic virus.

20. The genetic construct according to claim 18, wherein the genetic construct is for insertion into an inactivated thymidine kinase gene region of the oncolytic virus.

21. An anti-cancer adjuvant comprising the oncolytic virus according to any one of claims 1 to 11 as an active ingredient.

22. A method of preventing or treating cancer, comprising the step of administering to a subject a composition comprising the oncolytic virus according to any one of claims 1 to 11 as an active ingredient.

23. The use of the oncolytic virus according to any one of claims 1 to 11 or a composition comprising the oncolytic virus for the prevention or treatment of cancer.

24. The use of the oncolytic virus according to any one of claims 1 to 11 or a composition comprising the oncolytic virus for the preparation of a drug for preventing or treating cancer.
